(19)

Europäisches
Patentamt
European
Patent Office
Office européen
des brevets

(11) **EP 3 117 354 B1**

(12) **EUROPEAN PATENT SPECIFICATION**

(45) Date of publication and mention
of the grant of the patent:
**29.08.2018 Bulletin 2018/35**

(21) Application number: **15711867.0**

(22) Date of filing: **03.03.2015**

(51) Int Cl.:
***G06F 19/00*** *(2018.01)*

(86) International application number:
**PCT/IB2015/051530**

(87) International publication number:
**WO 2015/136406 (17.09.2015 Gazette 2015/37)**

(54) **OPTIMIZATION OF ALARM SETTINGS FOR ALARM CONSULTANCY USING ALARM REGENERATION**

OPTIMIERUNG VON ALARMEINSTELLUNGEN FÜR ALARMKONSULTATION MITTELS ALARMREGENERATION

OPTIMISATION DE RÉGLAGES D'ALARME POUR UNE CONSULTATION D'ALARME À L'AIDE D'UNE RÉGÉNÉRATION D'ALARME

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **14.03.2014 US 201461953126 P**

(43) Date of publication of application:
**18.01.2017 Bulletin 2017/03**

(73) Proprietor: **Koninklijke Philips N.V.**
**5656 AE Eindhoven (NL)**

(72) Inventors:
• **DE WAELE, Stijn**
**NL-5656 AE Eindhoven (NL)**

• **NIELSEN, Larry**
**NL-5656 AE Eindhoven (NL)**
• **YANG, Lin**
**NL-5656AE Eindhoven (NL)**

(74) Representative: **Ledeboer, Johannes Albertus**
**Philips Intellectual Property & Standards**
**High Tech Campus 5**
**5656 AE Eindhoven (NL)**

(56) References cited:
**WO-A1-2012/140547     US-A1- 2013 045 685**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

The present application relates generally to patient monitoring. It finds particular application in conjunction with optimization of alarm settings, and will be described with particular reference thereto. However, it is to be understood that it also finds application in other usage scenarios and is not necessarily limited to the aforementioned application.

Patient monitors contribute to patient safety by raising an alarm in the presence of abnormal vital signs that indicate patient deterioration. Several modalities have been introduced to routine intensive care unit (ICU) care throughout the second half of the 20th century, resulting in the rich monitoring landscape existing today. These modalities include electrocardiogram (ECG), peripheral capillary oxygen saturation (SpO2), blood pressure, and respiratory rate monitoring. With the availability of more monitoring modalities comes the challenge of proper alarm configuration to reduce false and clinically insignificant alarms, while retaining alarms of clinical significance, especially those associated with clinical events and episodes. This applies both to single parameter alarms and to more advanced multi-parameter alarms.

In 1994, a study found that over 94% of alarm soundings in a pediatric ICU may not be clinically important. Similar results have been found since in different studies throughout the years. For example, the ECRI Institute ranked alarm hazards as the second greatest health technology hazard in 2011 and the greatest health technology hazard in 2012 and 2013. In response to this broadly experienced hazard, The Joint Commission has initiated a patient safety goal on alarm management for 2014, requiring hospitals to improve their alarm management.

A proposed method for improving alarm management includes using multi-parameter alarm algorithms. Traditional alarm algorithms are single-parameter. Multi-parameter alarm algorithms offer greater sensitivity and specificity. An example of a multipath alarm algorithm is an alarm algorithm based on an early warning score (EWS) system or one of its variants. Another proposed method for improving alarm management includes performing large patient trials to determine appropriate default settings. However, this proposed method suffers from a knowledge gap in that there are no studies on the proper settings for alarm thresholds. Another proposed method for improving alarm management includes site-specific interventional trials for local optimization of alarm strategies.

With the large variation between hospitals, hospital wards, and hospital critical care units in patient population, nurse-to-patient ratio and both quality and quantity of monitoring equipment, it is expected that site-specific interventional trials for local optimization of alarm strategies will remain important as the other proposed methods are implemented. This is because the patient population and their vital signs characteristics varies significantly across units. The interventional trial is typically organized into three stages: pre-intervention; intervention; and post-intervention. In the pre-intervention stage, alarms for a set of patients are recorded, the most frequent alarms are identified, and a proposed alarm strategy (e.g., alarm threshold, alarm severity, etc.) is defined. In the intervention stage, the proposed alarm strategy is applied to a new set of patients. In the post-intervention stage, alarms for the new set of patients are recorded. The post- and pre-intervention alarm datasets are then compared to draw conclusions on the effectiveness and safety of the proposed alarm strategy.

A challenge with performing a site-specific interventional trial is that there is no accurate prediction for the impact of the proposed alarm strategy on alarm load, work flow, and patient safety. As such, the quality of the proposed new alarm strategy is limited. Further, the lack of a prediction regarding patient safety poses a risk to patient safety during the post-intervention stage.

Another challenge with performing a site-specific interventional trial is that the post-intervention stage is typically long. The proposed alarm strategy is applied to a new set of patients, which introduces a significant source of variance in estimating the reduction in alarm load. Therefore, a relatively long post-intervention stage is needed to draw statistically significant conclusions.

Another challenge with performing a site-specific interventional trial is expense. The post-intervention stage is expensive because of regulatory approval, training of personnel, and progress and safety monitoring. Also, definition of the proposed alarm strategy in the first-intervention stage is expensive because it requires physician hours to agree on new settings. Nonetheless, relative to the post-intervention stage, the pre-intervention stage is inexpensive and without impact on workflow.

US 2013/0045685 A1 discloses a medical sanitation device including a detector for detecting the physical presence of a clinician token within a detection area in the vicinity of the medical sanitation device. The clinician token may be indicative of the identity of a clinician. The medical sanitation device also includes a sanitation module configured to be used by the clinician to perform a sanitation task. Detection of a clinician in proximity to the medical sanitation device may be used to at least partially control access to, or operation of, a medical patient monitoring device

The present application provides a new and improved system and method which overcome these problems and others.

In accordance with one aspect, a system for optimization of alarm settings of a clinical alarm algorithm is provided as defined in claim 1.

In accordance with another aspect, a method for optimization of alarm settings of a clinical alarm algorithm is provided as defined in claim 9.

One advantage resides in an accurate prediction for the impact of a proposed alarm strategy.

Another advantage resides in improved patient safety.

Another advantage resides in reduced expense and duration compared to an interventional trial.

Still further advantages of the present invention will be appreciated to those of ordinary skill in the art upon reading and understand the following detailed description.

The invention may take form in various components and arrangements of components, and in various steps and arrangements of steps. The drawings are only for purposes of illustrating the preferred embodiments and are not to be construed as limiting the invention.

FIGURE 1 illustrates an information technology (IT) infrastructure of a medical institution, along with an alarm generation system.

FIGURE 2 illustrates a graph of alarm count as a function of encounter fraction.

FIGURE 3 illustrates a graph of alarm load in terms of alarms per bed per day as a function of alarm threshold.

FIGURE 4 illustrates a graph of percentage of alarms with durations greater than delay as a function of delay for various alarm thresholds.

FIGURE 5 illustrates a cumulative histogram describing the fraction of time an alarm threshold is met across a patient population for each of a plurality of heart rate bin.

FIGURE 6 illustrates a flowchart of alarm regeneration with an early warning score (EWS) system.

FIGURE 7 illustrates a method for optimization of alarm settings using alarm regeneration.

The present application discloses an approach for optimizing alarm settings. According to the approach, vital signs and context data are recorded over a period of time. Alarms are then regenerated based on the recorded data for several proposed alarm settings. The regeneration allows alarm load and related parameters, such as the work load impact and detection performance (i.e., sensitivity and specificity), to be accurately predicted for the specific patient population of the recorded data.

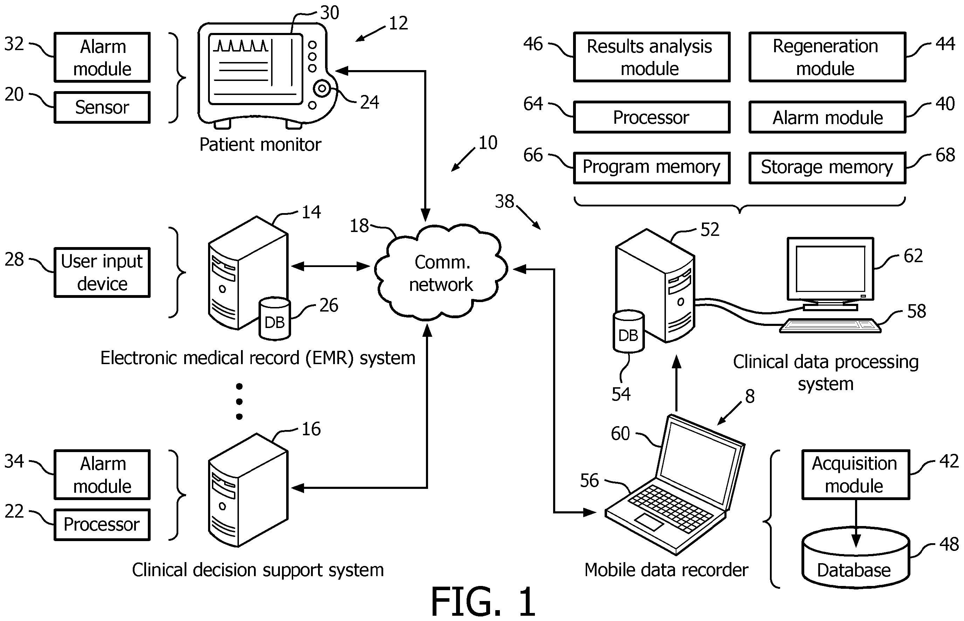

With reference to FIGURE 1, an information technology (IT) infrastructure **10** of a medical institution includes one or more sources **12, 14, 16** of clinical monitoring data (CMD) connected through a communications network **18.** The communications network **18** is typically a local area network (LAN), but other types of communications networks are contemplated. CMD is any data used by a clinical monitoring algorithm (CMA) to generate an alarm in response to detecting patient deterioration. Examples of CMD sources **12, 14, 16** include, but are not limited to, patient monitors **12,** nursing stations, mobile communications devices, patient information systems **14,** clinical decision support systems **16,** and so on.

The CMD sources **12, 14, 16** store or generate CMD for corresponding patients. The CMD for a patient includes data indicative of the physiological state of the patient. Data indicative of the physiological state of a patient includes data indicative of vital signs (e.g., heart rate, electrocardiogram (ECG), blood pressure, peripheral capillary oxygen saturation (SpO2), etc.), lab tests (e.g., creatine, blood urea nitrogen (BUN), alanine aminotransferase (ALT), etc.), physiological scores (e.g., early warning score (EWS), vital signs instability index (VIX), simplified acute physiology score (SAPS)-I, etc.), fluids, demographics (e.g., age, gender, etc.), alarms, and other variables relevant to the physiological state of the patient (e.g., diagnosis, intensive care unit (ICU) type, etc.).

A CMD source **12, 16** generating CMD is a CMD producer **12, 16**. A CMD producer **12, 16** typically generates CMD continuously or upon the happening of an event, such as a timer event, a user input event, and so on. Further, a CMD producer **12, 16** generates CMD automatically or manually. A CMD producer **12, 16** can generate CMD automatically by, for example, measuring a variable indicative of the physiological state of a patient with a sensor **20** or by calculating a value indicative the physiological state of the patient with a processor **22**. A CMD producer **12, 16** can generate CMD manually by, for example, receiving user input indicative of the physiological state of a patient from a clinician with a user input device **24.** Examples of CMD producers **12, 16** include, but are not limited to, patient monitors **12,** and clinical decision support systems **16.**

A CMD source **14** storing CMD is a CMD store **14.** A CMD source can be both a CMD producer **12, 16** and a CMD store **14.** A CMD store **14** stores CMD locally in a database **26.** The CMD stored can be received locally or remotely. For example, the CMD can be received remotely from other CMD sources **12, 16** or can be received locally from a user with a user input device **28.** Examples of CMD stores **14** include, but are not limited to, electronic medical record (EMR) systems **14,** departmental systems, and the like.

The CMD sources **12, 14, 16** and the alarm modules **32, 34** are each one of software, hardware, or a combination of the two. Where a CMD source **12, 14, 16** or alarm module **32, 34** includes or is software, the CMD source **12, 14, 16** or the alarm module **32, 34** typically includes or is associated with, respectively, at least one processor **22** and at least one program memory. The at least one processor **22** executes processor executable instructions on the at least one program memory to carry out the functionality of the CMD source **12, 14, 16** or the alarm module. Similarly, where a CMD source **12, 14, 16** or alarm module **32, 34** receives user input or displays data, the CMD source **12, 14, 16** typically includes or is associated with a user input device **24, 28** or a display device **30,** respectively.

Typically, the CMD sources **12, 14, 16** include at least one CMD producer **12, 16** of alarm data. A CMD producer **12, 16** of alarm data is associated with one or more patients and includes a clinical monitoring algorithm implemented in an alarm module **32, 34.** The clinical monitoring algorithm monitors the patients for patient deterioration based on received CMD. In response to detecting patient deterioration, the clinical monitoring algorithm generates an alarm. The clinical monitoring algorithm receives the CMD locally (e.g., by a user input device **24**) or remotely (e.g., from another CMD source **12, 14, 16**). Examples of CMD producers **12, 16** of alarm data include patient monitors **12,** clinical decision support systems **16,** and the like.

Clinical monitoring algorithms can be categorized by the way the algorithms are configured. In that vein, at least three different classes of clinical monitoring algorithms exist: tunable alarm algorithms, on/off alarm algorithms, and threshold alarm algorithms. Tunable alarm algorithms trigger an alarm when a non-vital sign (e.g., sleep apnea duration) exceeds a user-defined threshold. On/off alarm algorithms trigger an alarm when a binary physiological parameter (e.g., premature ventricular beat (PVC)) indicates an "on" state. Such on/off alarm algorithm may or may not have user-adjustable parameters. Threshold alarm algorithms trigger an alarm when a vital sign (e.g., heart rate) exceeds a user-defined threshold.

A threshold alarm algorithm can include filtering mechanisms to reduce the alarm rate. One such filtering mechanism is a delay mechanism in which a triggered alarm is suppressed if it lasts less than a user-defined number of seconds. Another such filtering mechanism is an inhibition mechanism in which a triggered alarm is suppressed if there was an alarm of the same type in the last user-defined number of seconds.

With continued reference to FIGURE 1, the IT infrastructure **10** further includes an alarm regeneration system **38.** The alarm regeneration system **38** optimizes alarm settings of a clinical monitoring algorithm for a user-defined patient population of the medical institution. The clinical monitoring algorithm is implemented by an alarm module **40** of the alarm regeneration system **38.** An alarm setting refers to a value of a user-defined parameter of the clinical monitoring algorithm or a related algorithm (e.g., an algorithm providing input to the clinical monitoring algorithm, such as a EWS algorithm). The user-defined parameters of a threshold alarm algorithm include, for example, threshold, delay and inhibition period. The user defined patient population can, for example, be a user-defined set of one or more medical units or wards within the medical institution. The clinical monitoring algorithm can be implemented within the CMD sources **12, 14, 16** of the IT infrastructure **10,** or within the alarm regeneration system **38.**

The alarm regeneration system **38** acquires and stores CMD, including vital signs and context data, required by the clinical monitoring algorithm for the user-defined patient population from the CMD sources **12, 14, 16** over a user-defined period of time (e.g., two weeks or six months). The alarm settings for the clinical monitoring algorithm are then optimized through alarm regeneration. More specifically, alarms are regenerated through application of the clinical monitoring algorithm to the stored CMD with several different alarm settings. The regeneration results are then assessed to accurately predict the effect that the different alarm settings have on alarm load and related parameters, such as the work load impact and detection performance (i.e., sensitivity and specificity), for the user-defined patient population. To carry out the foregoing, the alarm regeneration system **38** includes a plurality of modules: an acquisition module **42;** a regeneration module **44;** and a results analysis module **46.**

The acquisition module **42** acquires CMD from the CMD sources **12, 14, 16** over the communications network **18** for the user-defined period of time and stores the acquired data in a mobile database **48.** The CMD includes vital signs for the user-defined patient population and further context data required by the clinical monitoring algorithm. Context data includes, for example, data describing lab results and patient diagnosis. The CMD can further include one or more of: data describing triggered alarms; data regarding patient deterioration, such as recorded clinical events regarding patient deterioration (e.g., deterioration-related interventions) or patient care level increases; and data regarding recorded or estimated workload per alarm-triggered intervention. The acquisition module **42** acquires the CMD at a sufficient sample rate for the clinical monitoring algorithm, such as a one second sampling rate for vital signs.

Typically, the acquisition module **42** and the mobile database **48** are included within a mobile data recorder **50** of the alarm regeneration system **38.** Before beginning acquisition of CMD, the mobile data recorder **50** is installed at the medical institution. For example, the mobile data recorder is connected directly to the communications network **18,** or indirectly to the communications network **18** by way of another device or system. Once installed, the CMD is acquired by the acquisition module **42** and stored in the mobile database **48** for the user-defined period.

Upon conclusion of the acquisition, the mobile data recorder **50** is uninstalled from the medical institution and physically transported to a central data processing system **52** of the alarm generation system **38.** The central data processing system **52** is typically located remote from the medical institution. After removal of Protected Health Information, the central data processing system **52** then downloads the data to a central database **54** and locally processes the data in the central database **54** with the other modules **44, 46,** as discussed below. As an alternative to employing the central data processing system **52,** the mobile data recorder **50** can locally process the data in the mobile database **48** with the other modules **44, 46,** as discussed below.

Regardless of where the data acquired by the acquisition module **42** is processed, the regeneration modules **44** next coordinates with the alarm module **40** to regenerate alarms through application of the clinical monitoring algorithm to the acquired CMD for the user-defined patient population for each setting of a set of proposed alarm settings. This allows accurate prediction of alarm load and related parameters as a function of proposed alarm settings. Typically, the proposed alarm settings are applied without regard for modifications made to corresponding alarm setting parameters by clinicians during acquisition. However, in some embodiments, the proposed alarm settings are only used to the extent that no such modifications to the corresponding alarm setting parameters are made during acquisition.

A proposed alarm setting is typically generic to the entire user-defined patient population and determined before regeneration. However, a proposed alarm setting can alternatively be dependent upon the CMD and determined during regeneration. For example, a proposed alarm setting can be determined per patient based on CMD about the patient, such as diagnosis, age, or lab results. As another example, a proposed alarm setting can be determined per vital sign sample of a patient based upon current vital sign values of the patient.

The proposed alarm settings can be manually, automatically, or both automatically and manually defined for the alarm setting parameters of the clinical monitoring algorithm or a related algorithm. As to manual definition of the proposed alarm settings, a user input device **56, 58** can, for example, be employed to manually define the proposed alarm settings. This is advantageous for scenarios in which a clinician or a panel of clinicians determines proposed alarm settings. As to the automatic definition of the proposed alarm settings, the parameter space of each of the user-defined parameters of the clinical monitoring algorithm can, for example, be uniformly sampled. Alternatively, an algorithm can determine the propose alarm settings based on the CMD or feedback from the results analysis module **46.**

Although the acquisition is configured to acquire vital sign and context data, situations may arise where only alarm data is available. In such instances, approximate alarm regeneration can be performed. More specifically, approximate alarm regeneration can be performed by applying the proposed alarm thresholds to the extremes of the alarms. A characteristic of this technique is that the results show the combination effect of vital sign values and alarm settings during acquisition. This approximation is known to work well when the clinical monitoring algorithm is a pure threshold alarm algorithm (i.e., a threshold alarm algorithm without any filtering mechanisms).

The results analysis module **46** receives the regeneration results from the regeneration module **44** and graphically displays the regeneration results with a display device **60, 62** to a clinician for evaluation. The regeneration results can be displayed in graphs illustrating the predicted alarm displayed per alarm type and aggregated in different ways, such as per alarm severity, per modality, per shift, etc. Further, the regeneration results can be displayed in graphs illustrating the predicted alarm load or a related parameter (e.g., workload or alarm rate) as a function of one or more alarm setting parameters.

In addition to display the regeneration results, the results analysis module **46** can automatically evaluate the regeneration results with at least one processor **64** and displays the results of the evaluation with the display device **60, 62.** For example, true and false alarm rate can be assessed and the results displayed where patient deterioration data was acquired. Similarly, workload or alarm rate can be estimated and displayed based on the regenerated alarms. In some embodiments, the results analysis module **46** provides feedback to the regeneration module **44** based on the evaluation results until a set of one or more objectives are achieved. In providing the feedback, the proposed alarm settings are continuously modified or tuned until the set of objectives are achieved. The set of objectives can, for example, include a target workload or alarm rate.

In some embodiments, where the acquired CMD includes alarms, the results analysis module **46** estimates the recorded alarm rate and the regenerated alarm rates for proposed alarm settings. The estimates can then be graphically displayed to allow quantitative comparison. Alternatively, the estimate of the recorded alarms can be automatically compared to the estimates of the regenerated alarms and alarm settings with reductions in alarm rate exceeding a user-defined threshold can be displayed.

Alarm rate $r$ is defined as the expectation of an alarm count $c$ generated by a single patient divided by a time interval $t$:

$$r = \mathrm{E}\left[\frac{c}{t}\right], \qquad (1)$$

where $r$ is given in counts per patient per day. To arrive at the alarm rate as experienced by a clinician, the alarm rate $r$ is multiplied by the number of patients under care of the clinician. When partitioning the time interval $t$ into $N$ patient encounters, each $i$ with an encounter alarm rate $r_i$, encounter alarm count $c$, and an encounter duration (i.e., length of stay) $d_i$, the alarm rate $r$ can be re-written as:

$$r = \mathrm{E}\left[\frac{\sum c_i}{\sum d_i}\right] = \mathrm{E}\left[\frac{\frac{1}{N}\sum d_i r_i}{\frac{1}{N}\sum d_i}\right]. \qquad (2)$$

The most direct expression for alarm rate $r$ is in terms of the alert count $c_i$ (i.e., not normalized to an alarm rate). To express the alarm rate $r$ terms of the encounter alarm rate $r_i$, the encounter alarm rates $r_i$ are weighted proportionally by the encounter duration $d_i$. The practical implication is that reducing false alarm rate for a patient with a combination of a high alarm rate and a long length of stay results in the greatest reduction of alarm fatigue.

The alarm rate is estimated by dividing the total alarm count by the sum of encounter durations:

$$\hat{r} = \frac{\sum c_i}{\sum d_i}. \qquad (3)$$

This can be derived from Equation (2). Assuming a Gaussian distribution of the rate estimate, the 95% confidence interval for this estimate is given by $[\hat{r} - 1.96\hat{\sigma}, \hat{r} - 1.96\hat{\sigma}]$, where $\hat{\sigma}$ is the estimated standard deviation, taken here as the square root of the estimated variance $\hat{V}$.

A starting point for a variance estimator is the sample variance, defined as follows. All encounters are concatenated on the time axis. The time axis is split into equal partitions of length $t_\sigma$. The alarm rate estimate within partition $k$ is denoted $r_{t_\sigma,k}$, the number of partitions is $M$. The sample variance $\hat{V}_S$ is given by:

$$\hat{V}_S = \frac{1}{M} \sum_k \left( r_{t_\sigma,k} - \hat{r} \right)^2, \qquad (4)$$

which results in the variance estimate $\hat{V}^*$:

$$\hat{V}^* = \frac{1}{M^2} \sum_k \left( r_{t_\sigma,k} - \hat{r} \right)^2. \qquad (5)$$

Partition boundaries are not aligned with encounter boundaries. This complicates the calculation of $\hat{V}^*$, and also introduces dependencies between the samples. Therefore, each encounter is used as a sample, and its contribution is weighed according to the encounter duration, resulting in the following variance estimate $\hat{V}$:

$$\hat{V} = \frac{1}{N^2} \sum_k \left( r_{t_\sigma,k} - \hat{r} \right)^2. \qquad (6)$$

Further, in some embodiments, the results analysis module **46** determines and displays the distribution of alarm rate per encounter. As discussed above, each alarm is associated with an encounter. A useful approach to display the distribution includes plotting the alarm rate as a function of the encounter, where the encounters are ordered from high to low alarm rate. Expressed in basic statistical functions, this is the inverse survival function $s^{inv}$:

$$s^{inv}(p) = X : P(x > X) = p, \qquad (7)$$

where p is the encounter fraction, $P(x > X)$ is the survival function for the alarm rate $X$ (i.e., the probability that $x > X$). The survival function is the reverse of the cumulative histogram: $P(x > X) = 1 - P(x \leq X)$.

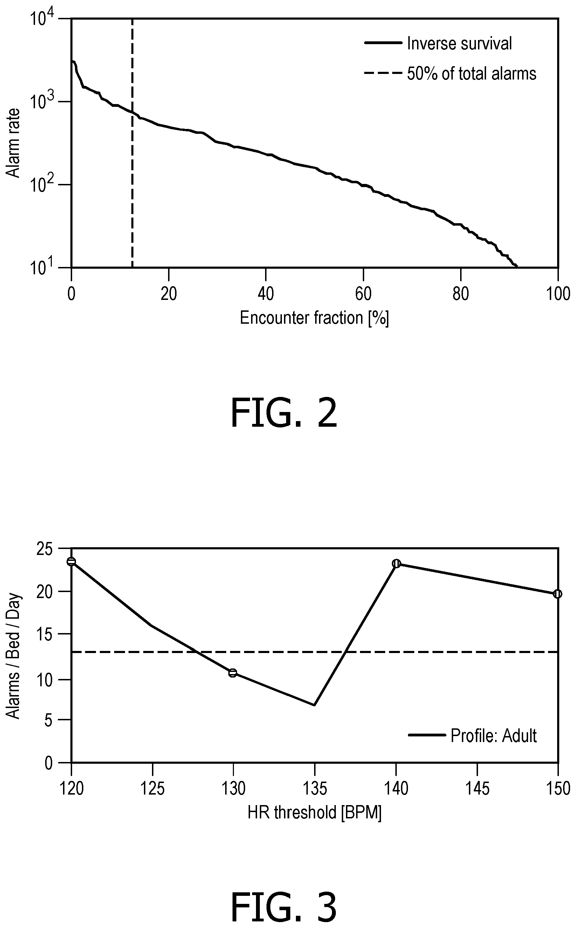

FIGURE 2 shows a graph of inverse survival function for alarm count per encounter. The alarm count distribution over patients can be characterized by the fraction of encounters that cause a given fraction of the total alarms. As shown, 50% of the alarms are caused by 12% of encounters with the highest alarm count. This statistic shows the potential alarm rate reduction that can be achieved by addressing the most alarming patients.

The regeneration results describe the predicted alarm load of the user-defined patient population. The results analysis module **46** can extend the regeneration results to predict the alarm load of a future patient population. More specifically, the CMD can be analyzed to determine a distribution of different patient types within the user-defined patient populations. Further, a distribution of different patient types can be defined for the future patient. The distributions can then be compared and the regeneration results can be reweighted. Patient types more prevalent in the future distribution are given more weight in the reweighted regeneration results than patient types more prevalent in the user-defined distribution. For example, if the user-defined patient population contains more geriatric patients than the average patient population, alarms for geriatric patients could be reduced to estimate regeneration results for an average patient population.

Advantageously, with alarm regeneration, the impact of a new alarm strategy can be quantified before new alarm settings are applied to new patients. More specifically, because the various settings are applied to the same patient population, the alarm load reduction can be calculated with great accuracy. Unlike methods where recorded alarms or alarm thresholds are used, the regeneration results are independent of the alarm settings that were active during the data collection.

As discussed above, the regeneration system **38** includes a plurality of modules **40, 42, 44, 46.** Each of the modules **40, 42, 44, 46** is one of software, hardware, or a combination of the two. Where a module **40, 42, 44, 46** includes or is software, the module **40, 42, 44, 46** typically includes or is associated with, respectively, at least one processor **64** and at least one program memory **66.** The at least one processor **64** executes processor executable instructions on the at least one program memory **66** to carry out the functionality of the module **40, 42, 44, 46.** Similarly, where a module **40, 42, 44, 46** receives user input or displays data, the module **40, 42, 44, 46** typically includes or is associated with a user input device **56, 58** or a display device **60, 62,** respectively.

Also, as discussed above, the regeneration system **38** includes at least one database **48, 54.** Each of the at least one database **48, 54** is one of software, hardware, or a combination of the two. Where a database **48, 54** includes or is software, the database **48, 54** typically includes or is associated with, respectively, at least one storage memory **68** storing the data of the database **48, 54.**

While the alarm regeneration system **36** is illustrated as including a mobile data recorder **50** and a central data processing system **38,** it is to be appreciated that components of the central data processing system **38** can be integrated with the mobile data recorder **50.** These components includes the alarm, regeneration and results analysis modules **40, 44, 46** and, in some embodiments, the at least one processor **64** and the program and storage memories **66, 68.** Advantageously, by integrating the components of the central data processing system **38** with the mobile data recorder **50,** the alarm settings of the medical institution can be optimized on site.

To illustrate operation of the regeneration system **38,** applicants hereafter describe two illustrative embodiments. According to the first illustrative embodiment, suppose the clinical monitoring algorithm is a threshold alarm algorithm with delay and inhibition filtering mechanisms. The threshold alarm algorithm can, for example, be employed by a patient monitor **12** of the IT infrastructure **10.** In applying the alarm regeneration system **38** to the first embodiment, the acquisition module **42** is employed to record vital signs for the user-defined patient population to the mobile database **48.** The vital signs are sampled at a rate to allow accurate regeneration with the threshold alarm algorithm.

Subsequent to acquisition, the regeneration module **44** is employed to perform alarm load prediction by applying the threshold alarm algorithm to the recorded vital signs with proposed alarm settings. Typically, the proposed alarm settings are generic to the entire user-defined patient population. Because the threshold alarm algorithm includes delay and inhibition filtering mechanism, there are three alarm setting parameters for which settings can be optimization: a threshold *Th*, a delay *D*, and an inhibition period *I*. As discussed above, the delay filtering mechanism suppresses a triggered alarm if it lasts less than the delay *D*, and an inhibition filtering mechanism suppresses a triggered alarm if there was an alarm of the same type in the immediately preceding inhibition period *I*.

To perform regeneration on a threshold alarm algorithm with delay and inhibition filtering, it is beneficial to perform the alarm regeneration in separate stages, rather than repeating the complete regeneration for each different setting. According to the first stage, proposed alarm settings for the threshold *Th* are evaluated. This includes, for each proposed alarm setting for the threshold, determining the time intervals over the user-defined period where the vital sign exceeds the proposed alarm setting. These times intervals represent regenerated alarms and are stored for subsequent use.

With the first stage complete, second and third stages derive the alarm load for different delays and inhibition periods, respectively, by filtering the regenerated alarms, without the need to reanalyze the vital sign over the entire user-defined period. Advantageously, this significantly accelerates the regeneration. According to the second stage, for each proposed alarm setting for the delay, the regenerated alarms where (*EndTime* - *AnnounceTime*) < *D* are removed. According to the third stage, for each proposed alarm setting for the inhibition period, the remaining regenerated alarms $n$ where $EndTime_n < EndTime_{n+1} + T$ are removed.

Upon completion of regeneration, the results analysis module **46** receives the foregoing results and displays the results to a clinician. FIGURES 3-5 provide example displays of results when the threshold alarm algorithm is applied to heart rate. The alarm threshold is in terms of beats per minute (BPM).

With reference to FIGURE 3, a graph of alarm load in terms of alarms per bed per day as a function of the alarm threshold is illustrated. The dashed line represents the median. The increase from threshold 135 to threshold 140 is because alarm thresholds of 140 and up are treated as critical level alarms (represented by vertically striped circles), which have no inhibition. Conversely, lower level, urgent alarms (represented by horizontally striped circles) have a default inhibition period of three minutes, which lowers the alarm load.

Suppose the alarm threshold is increased from 120 to 130, thereby increasing the lower level, urgent alarm threshold to 130. Further, suppose the difference between the critical level alarm threshold and the lower level, urgent alarm threshold is maintained at 20. In such a scenario, the critical level alarm threshold is further increased from 140 to 150. These changes result in the following alarm load reductions: lower level, urgent alarms are reduced from 22.8 to 10.1 (i.e., a 55% reduction); and critical level alarms are reduced from 22.5 to 18.7 (i.e., a 17% reduction).

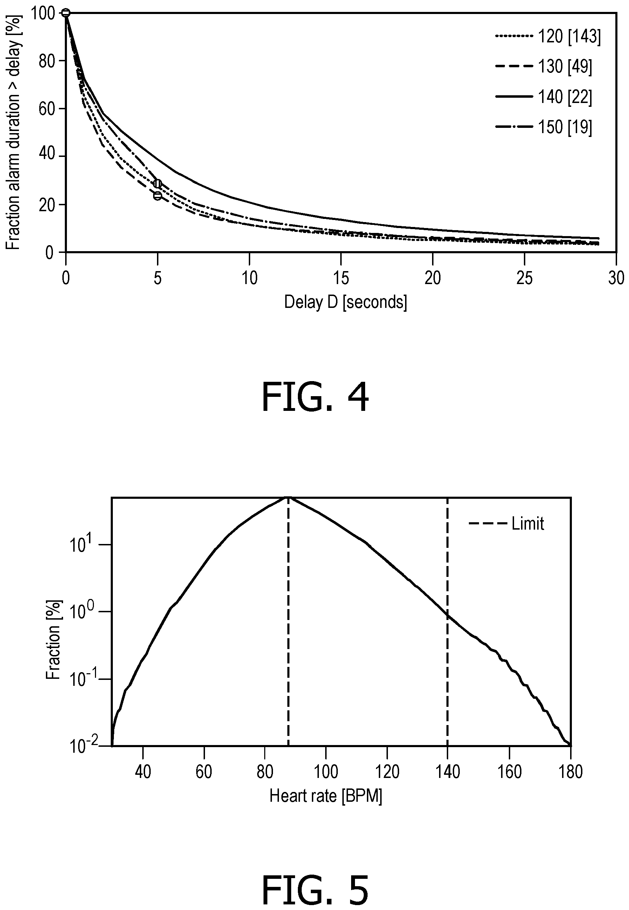

With reference to FIGURE 4, a graph of percentage of alarms with durations greater than delay as a function of delay for various alarm thresholds is illustrated. Suppose an increase in delay from zero to five seconds, assuming an alarm threshold of 130. This results in the following alarm load reductions: lower level, urgent alarms are reduced from 100% to 24.3% (i.e., a 75.7% reduction); and critical level alarms are reduced from 100% to 29.9% (i.e., a 70.1% reduction). Similar reductions can be achieved at the default alarm threshold of 120.

With reference to FIGURE 5, this figure illustrates a folded cumulative histogram describing the fraction of time an alarm threshold is met across a patient population for each of a plurality of heart rate bin. To facilitate visual evaluation of the distribution, the histogram $F$ is replaced by the survival function $1 - F$ for values greater than the median. For example, it can be determined that the HR is greater than 140 for 0.87% of time, or 31 seconds per hour. With a critical alarm without filtering mechanisms, this fraction is equal to the fraction of time the alarm sounds, and hence gives an indication of the alarm noise level caused by this alarm.

Referring back to FIGURE 1, according to the second illustrative embodiment, suppose the clinical monitoring algorithm is a threshold alarm algorithm employing a EWS system. EWS systems are commonly used by clinical monitoring algorithms in general wards. The EWS system calculates a EWS in real time by counting the contributions of several critical physiological parameters according to a EWS model. When the EWS passes one of a plurality of alarm thresholds, an alarm may be triggered and a certain clinical team may be required to respond depending upon the alarm threshold passed.

In applying the alarm regeneration system **38** to the second embodiment, the acquisition module **42** is employed to record the physiological parameters (e.g., level of consciousness) employed by the EWS system. The physiological data can, for example, be drawn from an EMR system **14** over the communications network **18**. Further, the physiological data can, for example, include data indicative of any of the following: vital signs; deterioration (e.g., an adverse event, a transfer to a higher level of care, etc.); demographics; lab results; medications; and any other data required by the EWS system. The recorded data is preferably from multiple years so variations between seasons or even years can be considered. However, if such data is limited, a chunk of data from each season is sufficient (e.g., a month-worth of data from each quarter of the year). If the EWS system is integrated in patient monitors **12,** high sampling-rate vital signs measured by the patient monitors needs to be similarly recorded at a high sampling rate.

After acquisition, the regeneration module **44** is employed for alarm load prediction by applying the threshold alarm algorithm and the EWS system to the recorded data with proposed alarm settings. The alarm setting parameters considered during alarm regeneration include alarm thresholds, as well as related parameters of the EWS system, such as the EWS model and the responsibilities of different clinical teams. The responsibilities define the correspondence between the alarm thresholds and the different clinical teams. Considering different EWS model settings allows alarm sensitivity and specificity to be tailored to different patient populations. Further, together the responsibilities, the EWS model and the responsibilities determine when and how often floor nurses should check the vital signs of a patient, when a physician or a rapid response team (RRT) should be called, whether a patient needs to be transferred to an ICU, etc.

The results analysis module **46** next evaluates the regeneration results, typically by estimating workload from the regeneration results. The workload estimation results can be displayed and typically include a list of statistics. For example, the list can include the number of alarms that have to be responded to by nurses (e.g., the number of alarms per nurse per day), the number of additional spot checks triggered by low-threshold alarms (e.g., the number of additional spot checks per nurse per day), the number of RRT calls triggered by high-threshold alarms (e.g., the number of RRT calls per day), the number of true positive alarms, the number of false alarms, sensitivity, and specificity, etc. Based on the workload estimation and operation data of the medical institution, the results analysis module **46** can further determine and display cost estimations and recommendations for operations of the medical institution, such as an estimation of seasonal fluctuation in patient populations, plans for staffing, or plans for device purchases or upgrades. The proposed alarm settings can be modified until the proper combination of alarm settings is found.

Extending the second embodiment, the proposed alarm settings are continuously refined or tuned until a target workload is achieved. More specifically, regeneration results are generated for an initial proposed alarm settings. These alarm settings are applied by the regeneration module **44** and the results analysis module **46** evaluates the regeneration results to determine whether the target workload is met. To the extent that the target workload is not met, feedback is provided to the regeneration module **44** so revised proposed alarm settings can be evaluated. This continues until the target workload is met. In this way, alarm settings can be optimized.

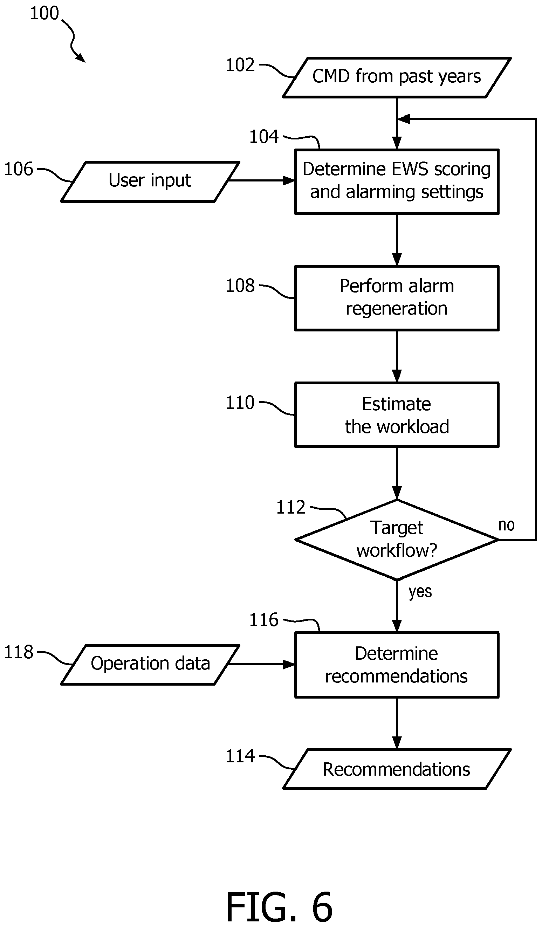

With reference to FIGURE 6, a flow chart **100** summarizes the second embodiment. As seen, patient data **102** from past years is acquired by the acquisition module **42.** Thereafter, initial EWS scoring and alarming settings are determined **104** by the regeneration module **44** based on user input **106,** typically from a clinician of the medical institution. Alarm regeneration is performed **108** on the settings by the regeneration module **44,** and workload is estimated **110** from the regeneration results by the results analysis module **46.** A determination **112** is made as to whether a target workload is achieved from the workload estimates. To the extent that the target is not achieved, the EWS scoring and alarming settings are revised or tuned, and regeneration and evaluation are performed again. This repeats until the target workload is achieved. Once the target workload is achieved, recommendations **114** are automatically determined **116** through application of the workload estimate to operation data **118** of the medical institution. The recommendations **114** are then displayed to clinicians.

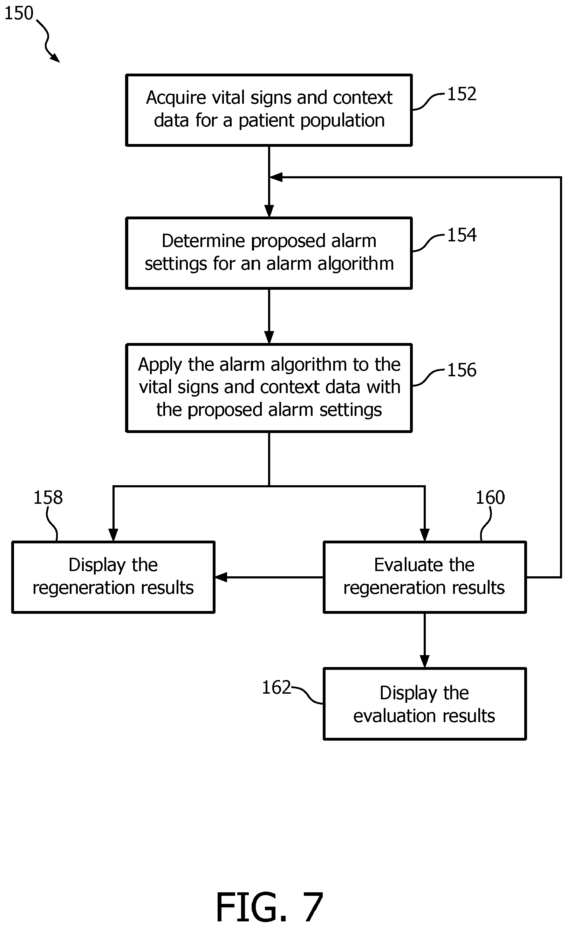

With reference to FIGURE 7, a method **150** for optimization of alarm settings of a clinical alarm algorithm is provided. The method **150** is performed by the modules **40, 42, 44, 46** of the alarm regeneration system **38.** According to the method **150,** CMD employed by the clinical alarm algorithm is acquired **152** over a user-defined period of time from the IT infrastructure **10.** The CMD is acquired for multiple patients of a user-defined patient population at a sufficient sample rate for the application to the clinical alarm algorithm. For example, vital sign data are acquired at a one second sample rate for the user-defined patient population over the user-defined period.

In addition to acquiring the CMD, proposed alarm settings are determined **154** for the configurable parameters of the clinical alarm algorithm and/or related algorithms. A related algorithm is, for example, a scoring system algorithm employed by the clinical alarm algorithm. The proposed alarm settings can be determined automatically, manually, or both automatically and manually. For example, proposed alarm settings can be determined automatically by sampling the parameter space and then the automatically determined settings can be modified by user input. With the determined proposed alarm settings and the acquired CMD, the clinical alarm algorithm is applied **156** to the CMD with the proposed alarm settings to predict alarm load and related parameters for different combinations of the proposed settings.

Where the clinical alarm algorithm is a threshold alarm algorithm, the parameters of the configurable parameters can include threshold, delay and inhibition period. The clinical alarm algorithm is applied **156** with settings for the threshold by, for each proposed setting of the threshold, determining a set of intervals during the user-defined period where the acquired CMD exceeds the proposed setting. These intervals correspond to alarms. The clinical alarm algorithm is applied with settings for the delay or the inhibition period by, for each proposed setting of the delay or the inhibition period, filtering the set of intervals based on the setting.

Where the clinical alarm algorithm is a threshold alarm algorithm employing a scoring system, the parameters of the configurable parameters can include parameters related to the scoring system. The scoring system can, for example, be the EWS system. Further, the parameters related to the scoring system can include, for example, the scoring model. The clinical alarm algorithm is applied **156** to the CMD with the proposed settings for the parameters related to the scoring system.

After performing regeneration, the regeneration results are displayed **158** or evaluated **160.** The evaluation can, for example, include determining whether the estimation achieves an objective, such as a target workload. The evaluation results can then be displayed **162.** Alternatively, based on the evaluation results, the proposed alarm settings can be revised or tuned and the foregoing can be performed again with the revised or tuned alarm settings. This then repeats until the objective is achieved. With the objective achieved, the regeneration results can be displayed.

In some embodiments, each of the constituent blocks **152, 154, 156, 158, 160, 162** illustrated in FIGURE 7 is a means for performing the corresponding functionality. The means for performing the corresponding functionality of each of the blocks **152, 154, 156, 158, 160, 162** includes one of: at least one processor programmed to perform the functionality; an application specific device configured to perform the functionality; and a combination of the at least one processor and an application specific devices coordination to perform the functionality.

As used herein, a memory includes any device or system storing data, such as a random access memory (RAM) or a read-only memory (ROM). Further, as used herein, a processor includes any device or system processing input device to produce output data, such as a microprocessor, a microcontroller, a graphic processing unit (GPU), an application-specific integrated circuit (ASIC), a FPGA, and the like; a controller includes any device or system controlling another device or system, and typically includes at least one processor; a user input device includes any device, such as a mouse or keyboard, allowing a user of the user input device to provide input to another device or system; and a display device includes any device for displaying data, such as a liquid crystal display (LCD) or a light emitting diode (LED) display.

**Claims**

1. A system **(38)** for optimization of alarm settings of a clinical alarm algorithm, said system **(38)** comprising:

   an acquisition module **(42)** configured to acquire clinical monitoring data, CMD, employed by the clinical alarm algorithm over a user-defined period of time;
   a regeneration module **(44)** configured to:

      determine proposed settings for one or more parameters of the clinical alarm algorithm; and
      apply the clinical monitoring algorithm to the acquired CMD with the proposed settings to determine regeneration results predicting alarm load for different combinations of the proposed settings,

wherein the clinical alarm algorithm is a threshold alarm algorithm,
wherein the one or more parameters include a threshold of the clinical alarm algorithm, and
wherein the regeneration module (44) is further configured to apply the clinical monitoring algorithm to the acquired CMD by, for each proposed setting of the threshold, determining a set of intervals during the user-defined period where the acquired CMD exceeds the proposed setting.

2. The system according to claim 1, wherein the acquisition module is further configured to:
acquire vital signs and context data as the CMD over a user-defined period of time.

3. The system (38) according to claim 1, wherein the one or more parameters further include a delay or inhibition period of the clinical alarm algorithm, and wherein the regeneration module (44) is further configured to:
apply the clinical monitoring algorithm to the acquired CMD by, for each proposed setting of the delay or inhibition period, filtering the set of intervals based on the setting.

4. The system (38) according to any one of claims 1-3, further including:
an alarm module (40) configured to monitor for patient deterioration through application of the clinical alarm algorithm to CMD, the alarm module (40) coordinating with the regeneration module (44) to predict alarm load.

5. The system (38) according to any one of claims 1-4, wherein the clinical alarm algorithm is a threshold alarm algorithm employing a scoring system, in particular an early warning score, EWS, system, wherein the one or more parameters includes parameters related to the scoring system, and wherein the regeneration module (44) is further configured to:
apply the clinical monitoring algorithm to the acquired CMD with proposed settings for the parameters related to the scoring system.

6. The system (38) according to any one of claims 1-5, further including:
a results analysis module (46) configured to display the regeneration results.

7. The system according to any one of claims 1-6, further including:
a results analysis module (46) configured to:

   evaluate the regeneration results to generate an estimation of workload; and
   provide feedback to the regeneration module (44) indicating the estimation does not achieve a target workload; and

wherein the regeneration module (44) is further configured to:

   revise the proposed settings based on the feedback; and
   apply the clinical monitoring algorithm to the acquired CMD with the revised proposed settings to determine revised regeneration results predicting alarm load for different combinations of the revised proposed settings.

8. The system (38) according to any one of claims 1-7, further including:

   a mobile data recorder (50) including the acquisition module (42), wherein the acquisition module (42) is configured to store the acquired CMD in a mobile database (48) of the mobile data recorder (50);
   a central data processing system (52) including the regeneration module (44), the central data processing system (52) configured to download the CMD from the mobile data recorder (50) for use by the regeneration module (44).

9. A computer-implemented method (150) for optimization of alarm settings of a clinical alarm algorithm, said method (150) comprising:

   acquiring (152) clinical monitoring data, CMD, employed by the clinical alarm algorithm over a user-defined period of time;
   determining (154) proposed settings for one or more parameters of the clinical alarm algorithm; and
   applying (156) the clinical monitoring algorithm to the acquired CMD with the proposed settings to determine regeneration results predicting alarm load for different combinations of the proposed settings,

wherein the clinical alarm algorithm is a threshold alarm algorithm,
wherein the one or more parameters include a threshold of the clinical alarm algorithm, and
wherein the method further includes applying (156) the clinical monitoring algorithm to the acquired CMD by, for each proposed setting of the threshold, determining a set of intervals during the user-defined period where the acquired CMD exceeds the proposed setting.

10. The method (150) according to claim 9, further including:
acquiring (152) vital signs and context data as the CMD over a user-defined period of time.

11. The method (150) according to claim 9, wherein the one or more parameters further include a delay or inhibition period of the clinical alarm algorithm, and wherein the method (150) further includes:
applying (156) the clinical monitoring algorithm to the acquired CMD by, for each proposed setting of the delay or inhibition period, filtering the set of intervals based on the setting.

12. The method (150) according to any one of claims 9-11, wherein the clinical alarm algorithm is a threshold alarm algorithm employing a scoring system, wherein the one or more parameters includes parameters related to the scoring system, and wherein the method (150) further includes:
applying (156) the clinical monitoring algorithm to the acquired CMD with proposed settings for the parameters related to the scoring system.

13. The method (150) according to any one of claims 9-12, further including:
displaying (158) the regeneration results.

14. The method (150) according to any one of claims 9-13, further including:

evaluating (160) the regeneration results to generate an estimation of workload and determine whether the estimation achieve a target workload;
revising (154) the proposed settings in response to the determination; and
applying (156) the clinical monitoring algorithm to the acquired CMD with the revised proposed settings to determine revised regeneration results predicting alarm load for different combinations of the revised proposed settings.

15. A non-transitory computer readable medium (66) carrying software which controls one or more processors (64) to perform the method (150) according to any one of claims 9-14.

**Patentansprüche**

1. System (38) zur Optimierung von Alarmeinstellungen eines klinischen Alarmalgorithmus, wobei das System (38) umfasst:

ein Erfassungsmodul (42), das so konfiguriert ist, dass es klinische • berwachungsdaten, CMD, erfasst, die von dem klinischen Alarmalgorithmus • ber eine benutzerdefinierte Zeitperiode verwendet werden;
ein Regenerationsmodul (44), das so konfiguriert ist, dass es:

vorgeschlagene Einstellungen f•r einen oder mehrere Parameter des klinischen Alarmalgorithmus ermittelt; und
den klinischen • berwachungsalgorithmus auf die erfassten CMD mit den vorgeschlagenen Einstellungen anwendet, um Regenerationsergebnisse zu ermitteln, die Alarmbelastung f•r verschiedene Kombinationen der vorgeschlagenen Einstellungen prognostizieren,

wobei der klinische Alarmalgorithmus ein Grenzwertalarmalgorithmus ist,
wobei der eine oder mehrere Parameter einen Schwellenwert des klinischen Alarmalgorithmus enthalten, und
wobei das Regenerationsmodul (44) weiterhin so konfiguriert ist, dass es den klinischen • berwachungsalgorithmus auf die erfassten CMD anwendet, indem es f•r jede vorgeschlagene Einstellung des Schwellenwertes einen Satz von Intervallen w, hrend der benutzerdefinierten Periode ermittelt, wo die erfassten CMD die vorgeschlagene Einstellung • berschreiten.

2. System nach Anspruch 1, wobei das Erfassungsmodul weiterhin so konfiguriert ist, dass es:
Vitalzeichen und Kontextdaten als die CMD • ber eine benutzerdefinierte Zeitperiode erfasst.

3. System (38) nach Anspruch 1, wobei der eine oder mehrere Parameter weiterhin eine Verz*f*gerungs- oder Inhibitionsperiode des klinischen Alarmalgorithmus enthalten, und wobei das Regenerationsmodul (44) weiterhin so konfiguriert ist, dass es:
den klinischen • berwachungsalgorithmus auf die erfassten CMD anwendet, indem es f•r jede vorgeschlagene Einstellung der Verz*f*gerungs- oder Inhibitionsperiode den Satz von Intervallen aufgrund der Einstellung filtert.

4. System (38) nach einem der Anspr•che 1-3, weiterhin umfassend:
ein Alarmmodul (40), das so konfiguriert ist, dass es durch Anwenden des klinischen Alarmalgorithmus auf CMD eine • berwachung im Hinblick auf Patientenverschlechterung vornimmt, wobei das Alarmmodul (40) mit dem Regenerationsmodul (44) koordiniert, um Alarmbelastung zu prognostizieren.

5. System (38) nach einem der Anspr•che 1-4, wobei der klinische Alarmalgorithmus ein Grenzwertalarmalgorithmus mit einem Scoring-System, vorzugsweise mit einem Fr•hwarn-Score-, EWS-, System, ist, wobei der eine oder mehrere Parameter auf das Scoring-System bezogene Parameter enthalten, und wobei das Regenerationsmodul (44) weiterhin so konfiguriert ist, dass es:
den klinischen • berwachungsalgorithmus auf die erfassten CMD mit vorgeschlagenen Einstellungen f•r die auf das Scoring-System bezogenen Parameter anwendet.

6. System (38) nach einem der Anspr•che 1-5, weiterhin umfassend:
ein Ergebnisanalysemodul (46), das so konfiguriert ist, dass es die Regenerationsergebnisse darstellt.

7. System nach einem der Anspr•che 1-6, weiterhin umfassend:
ein Ergebnisanalysemodul (46), das so konfiguriert ist, dass es:

die Regenerationsergebnisse auswertet, um eine Workload-Sch, tzung zu erzeugen; und
dem Regenrationsmodul (44) ein Feedback zuf•hrt, das signalisiert, dass die Sch, tzung keinen Ziel-Workload erreicht; und

wobei das Regenerationsmodul (44) weiterhin so konfiguriert ist, dass es:

die vorgeschlagenen Einstellungen aufgrund des Feedbacks revidiert; und
den klinischen • berwachungsalgorithmus auf die erfassten CMD mit den revidierten vorgeschlagenen Einstellungen anwendet, um revidierte Regenerationsergebnisse zu ermitteln, die Alarmbelastung f•r verschiedene Kombinationen der revidierten vorgeschlagenen Einstellungen prognostizieren.

8. System (38) nach einem der Anspr•che 1-7, weiterhin umfassend:

einen mobilen Datenrecorder (50), der das Erfassungsmodul (42) enth, lt, wobei das Erfassungsmodul (42) so konfiguriert ist, dass es die erfassten CMD in einer mobilen Datenbank (48) des mobilen Datenrecorders (50) speichert;
ein zentrales Datenverarbeitungssystem (52), das das Regenerationsmodul (44) enth, lt, wobei das zentrale Datenverarbeitungssystem (52) so konfiguriert ist, dass es die CMD von dem mobilen Datenrecorder (50) zur Verwendung durch das Regenerationsmodul (44) herunterl, dt.

9. Computerimplementiertes Verfahren (150) zur Optimierung von Alarmeinstellungen eines klinischen Alarmalgorithmus, wobei gem,,, dem Verfahren (150):

klinische • berwachungsdaten, CMD, erfasst werden (152), die von dem klinischen Alarmalgorithmus • ber eine benutzerdefinierte Zeitperiode verwendet werden;
vorgeschlagene Einstellungen f•r einen oder mehrere Parameter des klinischen Alarmalgorithmus ermittelt werden (154); und
der klinische • berwachungsalgorithmus auf die erfassten CMD mit den vorgeschlagenen Einstellungen anwendet wird (156), um Regenerationsergebnisse zu ermitteln, die Alarmbelastung f•r verschiedene Kombinationen der vorgeschlagenen Einstellungen prognostizieren,
wobei der klinische Alarmalgorithmus ein Grenzwertalarmalgorithmus ist,

wobei der eine oder mehrere Parameter einen Schwellenwert des klinischen Alarmalgorithmus enthalten, und wobei das Verfahren weiterhin das Anwenden (156) des klinischen • berwachungsalgorithmus auf die erfassten CMD beinhaltet, indem f•r jede vorgeschlagene Einstellung des Schwellenwertes ein Satz von Intervallen w, hrend der benutzerdefinierten Periode ermittelt wird, wo die erfassten CMD die vorgeschlagene Einstellung • berschreiten.

10. Verfahren (150) nach Anspruch 9, wonach weiterhin:
Vitalzeichen und Kontextdaten als die CMD • ber eine benutzerdefinierte Zeitperiode erfasst werden (152).

11. Verfahren (150) nach Anspruch 9, wobei der eine oder mehrere Parameter weiterhin eine Verz*f*gerungs- oder Inhibitionsperiode des klinischen Alarmalgorithmus enthalten, und wobei gem,,, dem Verfahren (150) weiterhin:
der klinische • berwachungsalgorithmus auf die erfassten CMD angewendet wird (156), indem f•r jede vorgeschlagene Einstellung der Verz*f*gerungs- oder Inhibitionsperiode der Satz von Intervallen aufgrund der Einstellung gefiltert wird.

12. Verfahren (150) nach einem der Anspr•che 9-11, wobei der klinische Alarmalgorithmus ein Grenzwertalarmalgorithmus mit einem Scoring-System ist, wobei der eine oder mehrere Parameter auf das Scoring-System bezogene Parameter enthalten, und wobei gfem,,, dem Verfahren (150) weiterhin:
der klinische • berwachungsalgorithmus auf die erfassten CMD mit vorgeschlagenen Einstellungen f•r die auf das Scoring-System bezogenen Parameter angewendet wird (156).

13. Verfahren (150) nach einem der Anspr* che 9-12, wonach weiterhin:
die Regenerationsergebnisse dargestellt werden (158).

14. Verfahren (150) nach einem der Anspr* che 9-13, wonach weiterhin:

die Regenerationsergebnisse ausgewertet werden (160), um eine Workload-Sch, tzung zu erzeugen und zu ermitteln, ob die Sch, tzung einen Ziel-Workload erreicht;
die vorgeschlagenen Einstellungen in Reaktion auf die Ermittlung revidiert werden (154); und
der klinische • berwachungsalgorithmus auf die erfassten CMD mit den revidierten vorgeschlagenen Einstellungen angewendet wird (156), um revidierte Regenerationsergebnisse zu ermitteln, die Alarmbelastung f•r verschiedene Kombinationen der revidierten vorgeschlagenen Einstellungen prognostizieren.

15. Nichttransitorisches computerlesbares Medium (66), auf dem Software gespeichert ist, die einen oder mehrere Prozessoren (64) so steuert, dass diese das Verfahren (150) nach einem der Anspr•che 9-14 ausfu•hren.

## Revendications

1. Syst•me (38) pour optimiser des r•glages d'alarme d'un algorithme d,alarme clinique, ledit syst•me (38) comprenant :

un module d'acquisition (42) configur• pour acqu•rir des donn•es de surveillance clinique CMD, employ• par l'algorithme d,alarme clinique sur une p•riode de temps d•finie par l'utilisateur;
un module de r•g•n•ration (44) configur• pour :

d•terminer des r•glages propos•s pour un ou plusieurs param•tres de l'algorithme d, alarme clinique ; et
appliquer l'algorithme de surveillance clinique aux CMD acquises avec les r•glages propos•s pour d•terminer des r•sultats de r•g•n•ration pr•disant une charge d'alarme pour diff•rentes combinaisons des r•glages propos•s,

dans lequel l'algorithme d'alarme clinique est un algorithme est un algorithme d'alarme de seuil,
dans lequel les un ou plusieurs param•tres comprennent un seuil de l'algorithme d, alarme clinique et
dans lequel le module de r•g•n•ration (44) est en outre configur• pour appliquer l'algorithme de surveillance clinique aux CMD acquises en d•terminant, pour chaque r•glage propos• du seuil, un ensemble d,intervalles au cours de la p•riode d•finie par l'utilisateurof les CMD acquis d•passent le r•glage propos•.

2. Syst•me selon la revendication 1, dans lequel le module d, acquisition est en outre configur• pour :
acqu•rir des signes vitaux et des donn•es de contexte comme CMD sur une p•riode de temps d•finie par l'utilisateur.

**3.** Syst•me (38) selon la revendication 1, dans lequel les un ou plusieurs param•tres comprennent en outre une p•riode de retard ou d, inhibition de l'algorithme d, alarme clinique et dans lequel le module de r•g•n•ration (44) est en outre configur• pour :

appliquer l'algorithme de surveillance clinique aux CMD acquises en filtrant, pour chaque r•glage propos• de la p•riode de retard ou d, inhibition, l'ensemble d, intervalles sur la base du r•glage.

**4.** Syst•me (38) selon l,une quelconque des revendications 1 ,, 3, comprenant en outre :
un module d,alarme (40) configur• pour surveiller la d•t•rioration d,un patient par application de l'algorithme d, alarme clinique aux CMD, le module d, alarme (40) se coordonnant avec le module de r•g•n•ration (44) pour pr•dire une charge d, alarme.

**5.** Syst•me (38) selon l,une quelconque des revendications 1 ,, 4, dans lequel 1, algorithme d, alarme clinique est un algorithme d, alarme de seuil employant un syst•me d'•valuation, en particulier un syst•me d'•valuation d, avertissement pr•coce EWS, dans lequel les un ou plusieurs param•tres comprennent des param•tres en rapport avec le syst•me d'•valuation et dans lequel le module de r•g•n•ration (44) est en outre configur• pour :
appliquer l'algorithme de surveillance clinique aux CMD acquises avec des r•glages propos•s pour les param•tres rapport•s au syst•me d'•valuation.

**6.** Syst•me (38) selon l,une quelconque des revendications 1 ,, 5, comprenant en outre :
un module d, analyse de r•sultats (46) configur• pour afficher les r•sultats de r•g•n•ration.

**7.** Syst•me selon l,une quelconque des revendications 1 ,, 6, comprenant en outre :

un module d, analyse de r•sultats (46) configur• pour :

•valuer les r•sultats de r•g•n•ration pour g•n•rer une estimation de la charge de travail ; et
fournir une r•troaction au module de r•g•n•ration (44) indiquant que l'estimation n, atteint pas une charge de travail cible ; et

dans lequel le module de r•g•n•ration (44) est en outre configur• pour :

revoir les r•glages propos•s sur la base de la r•troaction ; et
appliquer l'algorithme de surveillance clinique aux CMD acquises avec les r•glages propos•s r•vis•s afin de d•terminer des r•sultats de r•g•n•ration r•vis•s pr•disant une charge d, alarme pour diff•rentes combinaisons des r•glages propos•s r•vis•s.

**8.** Syst•me (38) selon l,une quelconque des revendications 1 ,, 7, comprenant en outre :

un enregistreur de donn•es mobile (50) comprenant le module d, acquisition (42), dans lequel le module d'acquisition (42) est configur• pour stocker les CMD acquises dans une base de donn•es mobile (48) de l'enregistreur de donn•es mobile (50) ;
un syst•me de traitement de donn•es central (52) comprenant le module de r•g•n•ration (44), le syst•me de traitement de donn•es central (52) •tant configur• pour t•l•charger les CMD issues de l'enregistreur de donn•es mobile (50) pour utilisation par le module de r•g•n•ration (44).

**9.** Proc•d•mis en ..uvre par ordinateur (150) pour l'optimisation de r•glages d, alarme d,un algorithme d'alarme clinique, ledit proc•d• (150) comprenant :

l'acquisition (152) de donn•es de surveillance clinique CMD employées par l'algorithme d'alarme clinique sur une p•riode de temps d•finie par l'utilisateur;
la d•termination (154) de r•glages propos•s pour un plusieurs param•tres de l'algorithme d, alarme clinique ; et
l'application (156) de l'algorithme de surveillance clinique aux CMD acquises avec les r•glages propos•s pour d•terminer des r•sultats de r•g•n•ration pr•disant une charge d'alarme pour diff•rentes combinaisons de r•glages propos•s,
dans lequel l'algorithme d, alarme clinique est un algorithme d, alarme de seuil,
dans lequel les un ou plusieurs param•tres comprennent un seuil de l'algorithme d, alarme clinique et
dans lequel le proc•d• comprend en outre l'application (156) de l'algorithme de surveillance clinique aux CMD acquises en d•terminant, pour chaque r•glage propos• du seuil, un ensemble d,intervalles au cours de la p•riode d* finie par l'utilisateurof les CMD acquises d•passent le r•glage propos•.

10. Proc•d•(150) selon la revendication 9, comprenant en outre :
l'acquisition (152) de signes vitaux et de donn•es de contexte comme CMD sur une p•riode de temps d•finie par l'utilisateur.

11. Proc•d•(150) selon la revendication 9, dans lequel les un ou plusieurs param•tres comprennent en outre une p•riode de retard ou d, inhibition de l'algorithme d, alarme clinique et dans lequel le proc•d• (150) comprend en outre :
l'application (156) de l'algorithme de surveillance clinique aux CMD acquises en filtrant, pour chaque r•glage propos• de la p•riode de retard ou d'inhibition, l,ensemble d'intervalles sur la base du r•glage.

12. Proc•d• (150) selon l,une quelconque des revendications 9 ,, 11, dans lequel l'algorithme d, alarme clinique est un algorithme d, alarme de seuil employant un syst•me d'•valuation, dans lequel les un ou plusieurs param•tres comprennent des param•tres rapport•s au syst•me d'•valuation et dans lequel le proc•d• (150) comprend en outre :
l'application (156) de l'algorithme de surveillance clinique aux CMD acquises avec des r•glages propos•s pour les param•tres rapport•s au syst•me d'•valuation.

13. Proc•d• (150) selon l,une quelconque des revendications 9,, 12, comprenant en outre :
l'affichage (158) des r•sultats de r•g•n•ration.

14. Proc•d• (150) selon l,une quelconque des revendications 9 ,, 13, comprenant en outre :

l'•valuation (160) des r•sultats de r•g•n•ration pour g•n•rer une estimation de charge de travail et d•terminer si l'estimation atteint une charge de travail cible ;
la r•vision (154) des r•glages propos•s en r•ponse ,, la d•termination ; et
l'application (156) de l'algorithme de surveillance clinique aux CMD acquises avec les r•glages propos•s r•vis•s pour d•terminer des r•sultats de r•g•n•ration r•vis•s pr•disant une charge d, alarme pour diff•rentes combinaisons des r•glages propos•s •vis•s.

15. Support non transitoire lisible par ordinateur (66) portant un logiciel qui r•gle un ou plusieurs processeurs (64) pour effectuer le proc•d• (150) selon l,une quelconque des revendications 9 ,, 14.

FIG. 1

# FIG. 2

# FIG. 3

FIG. 4

FIG. 5

100

102 — CMD from past years

106 — User input → 104 — Determine EWS scoring and alarming settings

108 — Perform alarm regeneration

110 — Estimate the workload

112 — Target workflow? —no

yes

118 — Operation data → 116 — Determine recommendations

114 — Recommendations

# FIG. 6

150

Acquire vital signs and context
data for a patient population ⌐152

Determine proposed alarm
settings for an alarm algorithm ⌐154

Apply the alarm algorithm to the
vital signs and context data with ⌐156
the proposed alarm settings

158

Display the
regeneration results

160

Evaluate the
regeneration results

162⌐ Display the
evaluation results

# FIG. 7

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20130045685 A1